# EUROPÄISCHE PATENTSCHRIFT

(11) **EP 1 223 867 B1**
(45) Veröffentlichungstag und Bekanntmachung des Hinweises auf die Patenterteilung: **28.02.2007**
(21) Anmeldenummer: 00969504.0
(22) Anmeldetag: 13.10.2000
(51) Int. Cl.: A61B 10/00

(54) **ALLERGENSCHEIBENDOSE**
LIDDED CASE FOR ALLERGEN DISKS
BOITE POUR DISQUES D'ALLERGENES

(30) Priorität: 26.10.1999 DE 19951420
(43) Veröffentlichungstag der Anmeldung: 24.07.2002
(73) Patentinhaber: Merck Patent GmbH, 64293 Darmstadt (DE)
(72) Erfinder: PETER, Werner, 21493 Schwarzenbek (DE); KRAUSE, Reinhard, 21465 Reinbek (DE)
(86) Internationale Anmeldenummer: PCT/EP2000/010119
(87) Internationale Veröffentlichungsnummer: WO 2001/030241

(56) Entgegenhaltungen:
- EP-A- 0 513 614
- DE-U- 8 907 465
- GB-A- 2 112 755
- US-A- 4 439 319

## Beschreibung

Die Erfindung betrifft eine Vorrichtung zur Verpackung und Lagerung von Allergenscheiben.

Neben anderen Reagenzien werden bei dem laborchemischen Nachweis von Immunglobulin E oder G Allergenscheiben eingesetzt. Dies sind runde oder vieleckige flache Scheiben aus Papier, modifizierter Zellulose, Kunststoff oder ähnlichen Werkstoffen. Teilweise sind die Allergenscheiben auch durch zentrale Ausstanzungen ringförmig ausgebildet. Die Scheiben besitzen im allgemeinen einen Durchmesser von ca. 0,5 bis 1 cm und eine Dicke von ca. 0,1 bis 1 mm. Eine Vielzahl derartiger Allergenscheiben ist im Handel erhältlich.

Die Allergenscheiben sind kovalent oder adsorptiv mit entsprechenden Antigenen beschichtet, an die Antikörper z.B. aus Blutserum binden können und in nachgeschalteten Reaktionen nachgewiesen werden können. Bei der Bestimmung von Immunglobulin E oder G bildet die Allergenscheibe beispielsweise die Festphase in einem Enzymimmunoassay.

Aufgrund der Art ihrer Anwendung werden Allergenscheiben in vielen Labors in großer Anzahl eingesetzt. Da die Allergenscheiben äußerlich nicht zu unterscheiden sind, müssen sie in einem Verpackungssystem gelagert werden, das einen leichten Zugriff ermöglicht und das Verwechslungen ausschließt. Zudem müssen sie bei der Lagerung feucht gehalten werden.

Bislang werden Allergenscheiben in Glasflaschen mit Pufferlösung oder in den folgenden Arten von Kunststoffdosen gelagert:
1. Kunststoffkassette mit einem in Pufferlösung getauchten Schwamm
   Die Allergenscheiben sind sechseckig geformt und werden in einer Fläche angeboten, wobei sie durch Perforation an Sollreißstellen separiert werden können. Die so verpackten Allergenscheiben können nur schwer aus der Kassette entnommen werden. Zudem können die Ränder der Allergenscheiben außerhalb der Perforation ausreißen.
2. Dose mit Hartschaum (DE 89 07 465 U)
   Der Boden der Dose ist mit einem Puffer-getränkten Hartschaum belegt, der vertikale Einkerbungen aufweist. In diese Einkerbungen werden die Allergenscheiben vertikal gesteckt. Nachteilig ist hierbei, daß die Allergenscheiben sehr intensiv mit dem Hartschaum in Berührung kommen. Dabei bleiben leicht Hartschaumpartikel an den Allergenscheiben haften und stören die weitere Auswertung.
3. Kunststoffkassette mit eingesetztem Gitter
   Der Boden dieser Kassette ist mit einem puffergetränkten Schwamm belegt. Darauf befindet sich ein Kunststoffgitter. In die entstehenden Aussparungen kann jeweils eine Allergenscheibe flach auf den Schwamm gelegt werden. Nachteil dieser Kassette ist der große Platzbedarf, da die Allergenscheiben horizontal gelagert werden. Zudem können sie mit einer Pinzette nur schwer entnommen werden. Es besteht die Gefahr, daß einzelne Scheiben in benachbarte Kammern verrutschen.

Somit weisen alle bekannten Vorrichtungen erhebliche Nachteile auf, die einer zügigen Durchführung von Tests mit Allergenscheiben entgegenstehen.

Aufgabe der vorliegenden Erfindung war es daher, eine Vorrichtung zur Verpackung und Lagerung von Allergenscheiben zu entwickeln, die eine einfache Handhabung, eine platzsparende Anordnung der Allergenscheiben und eine Lagerung ohne Verwechslungsgefahr ermöglicht.

Es wurde gefunden, daß die oben genannten Anforderungen von der erfindungsgemäßen Klappdeckeldose erfüllt werden. Boden und Deckelteil der Dose sind bevorzugt untrennbar verbunden, so daß die Deckel verschiedener Dosen nicht vertauscht werden können. Boden und Deckelteil sind mit einem Schwamm belegt, so daß die Allergenscheiben gleichmäßig feucht bleiben. Auf den unteren Schwamm ist ein Rastergitter aufgelegt oder befestigt, das Kavitäten aufweist, die nach unten leicht konisch zulaufen. Auf diese Weise sitzen die Allergenscheiben vertikal und sehr stabil in den Kavitäten. Sie können nicht verrutschen. Die Entnahme mit einer Pinzette ist einfach.

Gegenstand der Erfindung ist daher eine Vorrichtung zur vertikalen Aufbewahrung von Allergenscheiben bestehend aus einem Bodenteil und einem Deckelteil, dadurch gekennzeichnet, daß
a) Boden- und Deckelteil mit einem Schwamm bedeckt sind;
b) auf das Bodenteil ein Rastergitter mit nach unten sich konisch verengenden Kavitäten aufgesetzt ist.

Bevorzugte Ausführungsform ist eine Vorrichtung, bei der Boden- und Deckelteil miteinander verbunden sind.

Bevorzugte Ausführungsform ist eine Vorrichtung, bei der Boden- und Deckelteil mit Scharnieren verbunden sind.

Bevorzugte Ausführungsform ist weiterhin eine Vorrichtung, die Schwämme aus Zellulose mit Naturfaserverstärkung enthält.

Bevorzugte Ausführungsform ist auch eine Vorrichtung deren Deckel- und/oder Bodenteil mit Dichtlippen versehen ist.
Abbildung 1 zeigt eine Aufsicht auf eine erfindungsgemäße Vorrichtung.
Abbildung 2 zeigt eine Seitenansicht.
In Abbildung 3 ist eine geschlossene erfindungsgemäße Dose gezeigt.

Die Abbildungen 4, 5 und 6 zeigen die Ergebnisse von Lagerversuchen von Allergenscheiben in verschiedenen Verpackungsformen.

Kernstück der erfindungsgemäßen Vorrichtung oder Dose ist das Gitterraster. Es erzeugt Kavitäten, die bevorzugterweise quadratisch oder rautenförmig ausgebildet sind und sich nach unten hin konisch verengen. Auf diese Weise können Allergenscheiben entlang der Diagonale der Kavitäten eingebracht werden und sinken in die Kavität bis sie von den nach unten enger zusammenlaufenden Wänden aufgehalten werden oder den Boden berühren. Durch diese Anordnung können die Allergenscheiben nicht verrutschen oder in benachbarte Kavitäten gelangen. Zudem können die Scheiben durch ihre vertikale Anordnung in der Dose leicht mit z.B. einer Pinzette entnommen werden.

Die Größe der Kavitäten kann je nach Größe der Allergenscheiben variiert werden. Für die meisten Allergenscheiben eignen sich Kavitäten zwischen 4x4 mm und 5x5 mm. In einer bevorzugten Ausführungsform wird ein 27x27x27 mm Rastergitter mit 25 Kavitäten von je 4,45x4,45 mm (Diagonale 6,3 mm) verwendet, die sich nach unten hin bis auf 4,25x4,25 mm (Diagonale 6 mm) verengen. Zur Lagerung einer größeren oder kleineren Anzahl von Allergenscheiben in einer Dose kann die Anzahl der Kavitäten beliebig verändert werden. Genauso kann die Größe und Verengung der Kavitäten an die Größe der Allergenscheiben angepasst werden.

In einer bevorzugten Ausführungsform sind Boden- und Deckelteil der Dose fest miteinander verbunden, damit nach dem Öffnen der Dose nicht ein falscher Deckel aufgesetzt werden kann und es zu Verwechslungen kommt, da Deckel verschiedener Dosen vertauscht werden. Die beiden Teile werden typischerweise über Scharniere oder sonstige übliche Verbindungen, wie beispielsweise bei einer aus einem Teil gefertigten Klappdeckeldose, zusammengehalten. Dies sind beispielsweise flexible Scharnierbügel oder andere Konstruktionen, typischerweise aus Kunststoff. Dem Fachmann sind verschiedene Ausführungsformen bekannt.

Boden- und Deckelteil der Dose sind mit einem Schwamm bedeckt, der mit entsprechenden Pufferlösungen getränkt werden kann. Auf diese Weise werden die Allergenscheiben ausreichend feucht gehalten, ohne daß sie den Schwamm großflächig berühren. Weiterhin verhindert der doppelte Schwamm-Einsatz Kondensationseffekte am Gehäuse und das Verrutschen oder Verschieben der Allergenscheiben.

Die Schwämme sind typischerweise den Maßen von Boden- bzw. Deckelteil angepaßt und bedecken sie vollständig. Die Schwämme oder Fließe bestehen bevorzugterweise aus hydrophilen, natürlichen oder synthetischen Stoffen, wie z.B. Zellulose, Filz oder Latexschaum, besonders bevorzugt aus pigmentierter Zellulose mit Naturfaserverstärkung. Durch ihre Fähigkeit, Flüssigkeit aufzusaugen, speichern die Schwämme genug Flüssigkeit, um die Allergenscheiben auch ohne weitere Flüssigkeitsschichten in den Dosen feucht zu halten. Es entstehen Lagerbedingungen nach dem Prinzip der "feuchten Kammer".

In einer bevorzugten Ausführungsform sind Deckel- und/oder Bodenteil, besonders bevorzugt der Deckel, mit einer Dichtlippe versehen. Auf diese Weise kann die erfindungsgemäße Dose dicht verschlossen werden und die Allergenscheiben bleiben lange feucht.

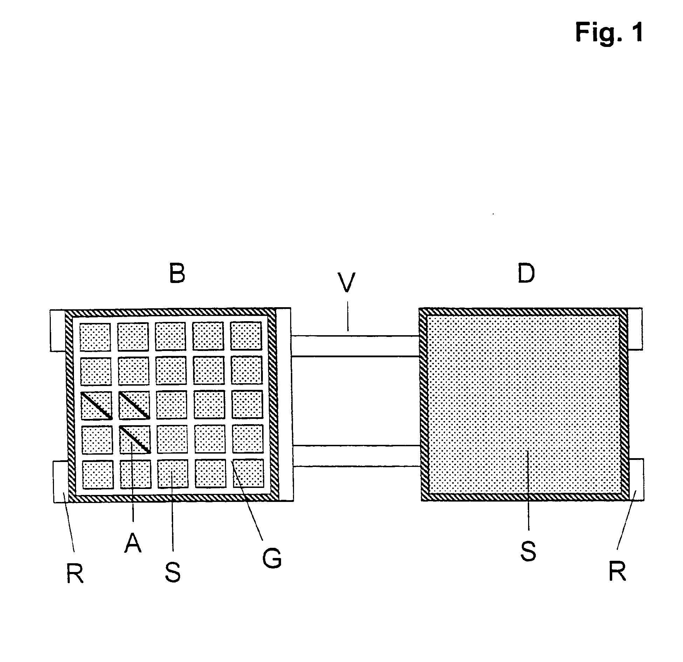

Abbildung 1 zeigt eine Aufsicht auf eine geöffnete erfindungsgemäße Dose. Bodenteil B und Deckelteil D sind jeweils mit einem Schwamm S bedeckt und über zwei Scharnierbügel V verbunden. Auf den Schwamm des Bodenteils ist ein Rastergitter G aufgesetzt. Es besitzt sich nach unten verengende Kavitäten, in die Allergenscheiben A diagonal eingesetzt werden können. Dadurch sind die Allergenscheiben vertikal angeordnet, sie können in den Kavitäten nicht verrutschen, berühren aber dennoch nicht mit ihren flachen Seiten die Wände der Kavitäten oder den Schwamm. Die Dose wird mittels üblicher Verschlüsse R an Deckel- und Bodenteil nach dem Zuklappen geschlossen gehalten.

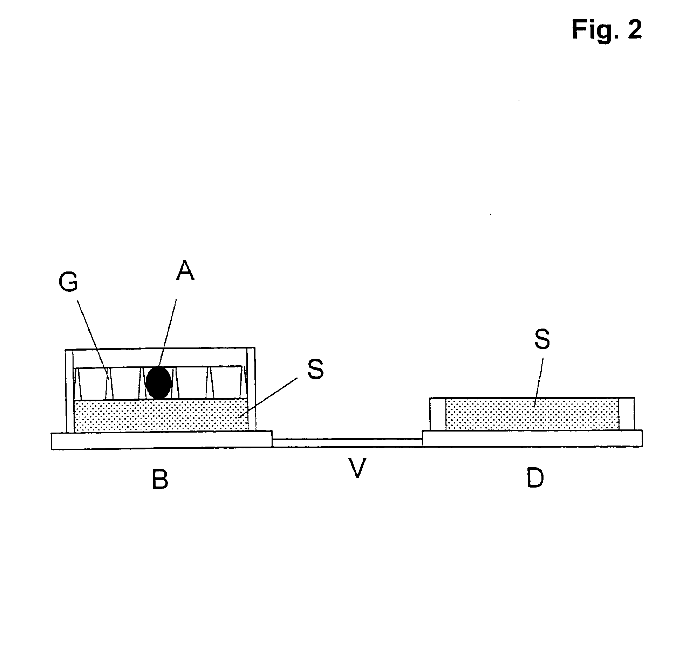

Abbildung 2 zeigt die Seitenansicht einer geöffneten Dose. Die Streben des Gitterrasters werden nach unten hin breiter und erzeugen so konisch zulaufende Kavitäten. Die eingesetzten Allergenscheiben gleiten je nach Größe in die Kavitäten bis sie auf den Schwamm am Boden treffen oder bis sie von den Wänden der Kavitäten aufgehalten werden.

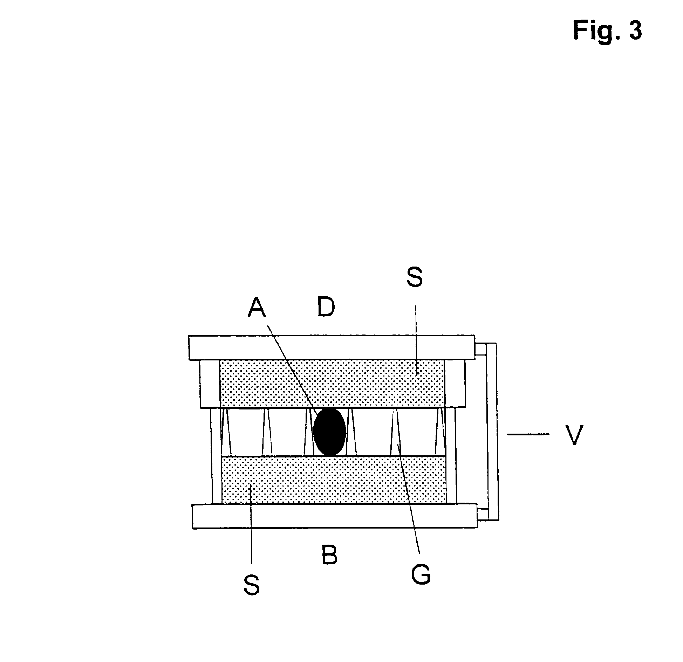

Abbildung 3 zeigt eine erfindungsgemäße Dose in geschlossenem Zustand. Die Allergenscheiben sind von den feuchten Schwämmen umgeben, ohne diese großflächig zu berühren.

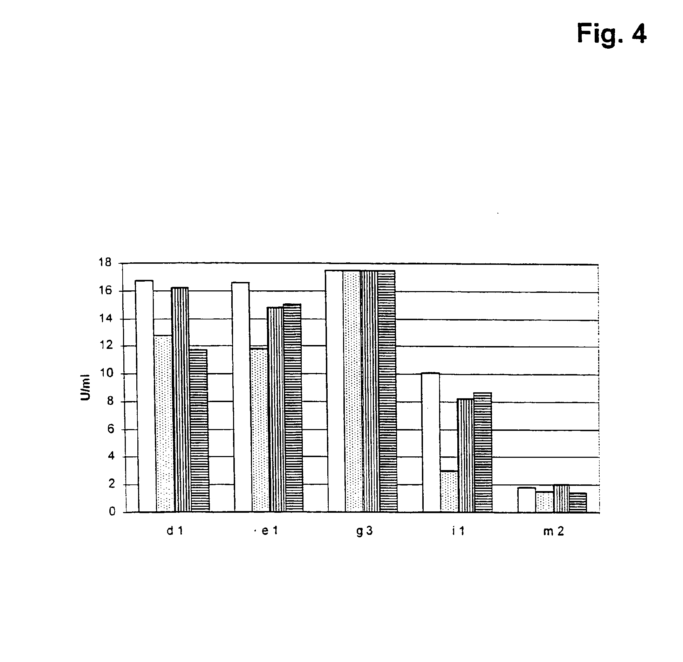

Die Abbildungen 4, 5 und 6 sind in dem Vergleichsbeispiel näher erläutert.

Somit werden durch die erfindungsgemäße Dose die eingangs genannten Nachteile beseitigt:
- Die Allergenscheiben können schnell und problemlos mit einer Pinzette entnommen werden. Sie können nicht in der Dose ankleben.
- Durch ein dicht schließendes Gehäuse und feuchte Schwämme an beiden Teilen der Dose werden die Allergenscheiben gleichmäßig befeuchtet.
- Durch die vertikale Lagerung in den Kavitäten berühren die Allergenscheiben den Schaum fast nicht, so daß keine Schaumpartikel an ihnen haften bleiben können.
- Auch unter Transportbedingungen oder bei Umkippen bleiben die Allergenscheiben fest am Platz.
- Die bevorzugte Ausführungsform der erfindungsgemäßen Dose, in der Boden- und Deckelteil verbunden sind, ist einfach zu öffnen und zu schließen, ohne daß der Deckel verwechselt werden kann.

Auch ohne weitere Ausführungen wird davon ausgegangen, daß ein Fachmann die obige Beschreibung im weitesten Umfang nutzen kann. Die bevorzugten Ausführungsformen und Beispiele sind deswegen lediglich als beschreibende, keineswegs als in irgendeiner Weise limitierende Offenbarung aufzufassen.

Die vollständige Offenbarung aller vor- und nachstehend aufgeführten Anmeldungen, Patente und Veröffentlichungen ist durch Bezugnahme in diese Anmeldung eingeführt.

### Vergleichsbeispiel

Die Abbildungen 4, 5 und 6 zeigen die Ergebnisse von Lagerversuchen von Allergenscheiben in verschiedenen Verpackungsformen. Untersucht wurde die Aktivität von Allergenscheiben mit unterschiedlichen Allergenen (d1, e1, g3, i1, m2) nach Lagerung unter verschiedenen Bedingungen. Folgende Verpackungsformen wurden gewählt:
1. Anfangsaktivität
   Zum Vergleich wurde die Anfangsaktivität der Allergenscheiben direkt nach der Produktion bestimmt.
2. Stand der Technik
   Die Allergenscheiben wurden in einer aus dem Stand der Technik (nach DE 89 07 465 U) bekannten Allergenscheibendose gelagert.
3. erfindungsgemäß
   Die Allergenscheiben wurden in einer erfindungsgemäßen Allergenscheibendose gelagert, wie sie in den Abbildungen 1 bis 3 dargestellt ist.
4. trocken
   Die Verpackung besteht aus einem geschlossenen Glasfläschchen ohne Feuchtkissen.

Die oben aufgeführten vier Verpackungsformen sind in den Abbildungen 4 bis 6 durch Balken mit folgenden Kennungen dargestellt:

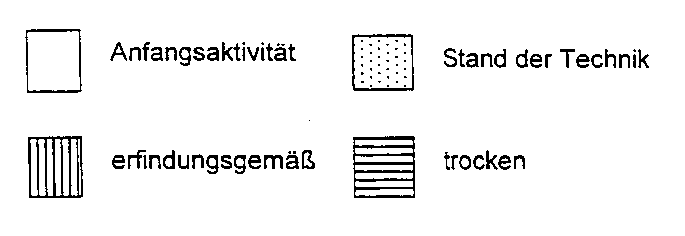

Die Aktivität der Allergenscheiben wurde nach bekannten Methoden mittels ELISA durch Bestimmung der Bindung von IgE an die jeweiligen Allergenscheiben bestimmt. Die Auswertung (in U/ml) erfolgte an einer parallel erarbeiteten Referenzkurve mit bekannten Konzentrationen von allergenspezifischem IgE.
Abbildung 4 zeigt die Aktivität der Allergenscheiben nach 32 Wochen Lagerung bei 2-8°C.

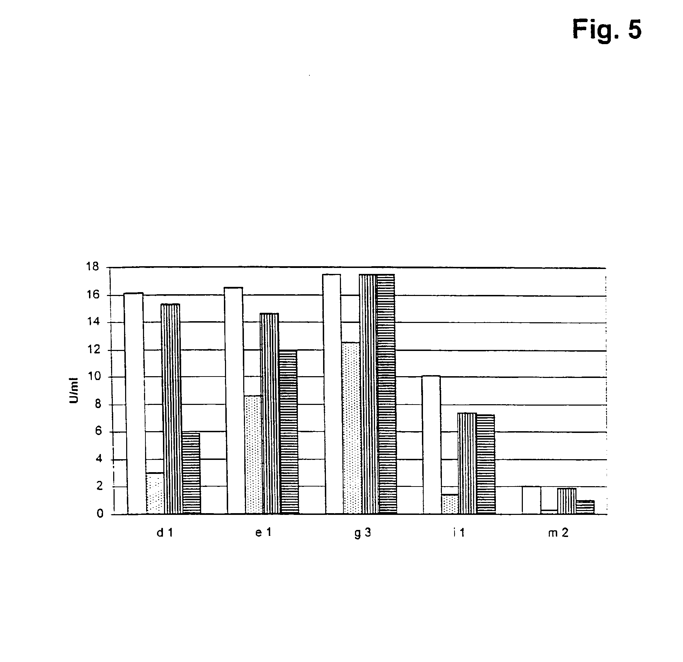

Abbildung 5 zeigt die Aktivität der Allergenscheiben nach 32 Wochen Lagerung bei 25°C.

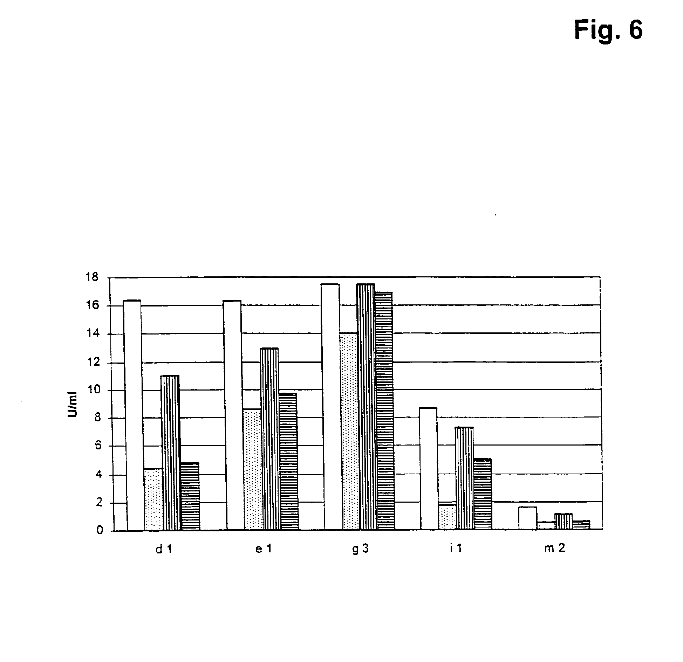

Abbildung 6 zeigt die Aktivität der Allergenscheiben nach 8 Wochen Lagerung bei 36°C.

Die Ergebnisse zeigen, daß die erfindungsgemäße Allergenscheibendose im Vergleich mit den anderen Verpackungen schonendere Lagerbedingungen schafft, die die Aktivität der Allergenscheiben lange aufrechterhalten.

## Patentansprüche

1. Vorrichtung zur vertikalen Aufbewahrung von Allergenscheiben bestehend aus einem mit einem Schwamm bedeckten Bodenteil (B) und einem Deckelteil (D), **dadurch gekennzeichnet, daß**
a) auch das Deckelteil mit einem Schwamm (S) bedeckt ist;
b) auf das Bodenteil ein Rastergitter (G) mit sich nach unten hin konisch verengenden Kavitäten aufgesetzt ist.

2. Vorrichtung nach Anspruch 1, **dadurch gekennzeichnet, daß** Boden- und Deckelteil miteinander verbunden sind.

3. Vorrichtung nach Anspruch 1 oder 2, **dadurch gekennzeichnet, daß** Boden- und Deckelteil mit Scharnierbügeln (V) verbunden sind.

4. Vorrichtung nach einem der Ansprüche 1 bis 3, **dadurch gekennzeichnet, daß** Boden- und Deckelteil mit Zelluloseschwämmen mit Naturfaserverstärkung bedeckt sind.

5. Vorrichtung nach einem der Ansprüche 1 bis 4, **dadurch gekennzeichnet, daß** Deckel- und/oder Bodenteil mit Dichtlippen versehen sind.

## Claims

1. Device for the vertical storage of allergen discs, consisting of a base part (B), which is covered with a sponge, and a lid part (D), **characterised in that**
a) the lid part is also covered with a sponge;
b) a grid (G) with cavities narrowing conically in the downward direction is placed on the base part.

2. Device according to Claim 1, **characterised in that** the base part and lid part are connected to one another.

3. Device according to Claim 1 or 2, **characterised in that** the base part and lid part are connected by hinge straps (V).

4. Device according to one of Claims 1 to 3, **characterised in that** the base part and lid part are covered with cellulose sponges with natural fibre reinforcement.

5. Device according to one of Claims 1 to 4, **characterised in that** the lid part and/or base part are provided with sealing lips.

## Revendications

1. Dispositif pour le stockage vertical de disques d'allergènes, constitué par une partie de base (B), laquelle est recouverte d'une éponge, et par une partie de couvercle (D), **caractérisé en ce que** :
a) la partie de couvercle est également recouverte d'une éponge ;
b) une grille (G) avec des cavités se rétrécissant de façon conique suivant la direction vers le bas est placée sur la partie de base.

2. Dispositif selon la revendication 1, **caractérisé en ce que** la partie de base et la partie de couvercle sont connectées l'une à l'autre.

3. Dispositif selon la revendication 1 ou 2, **caractérisé en ce que** la partie de base et la partie de couvercle sont connectées par des attaches en charnière (V).

4. Dispositif selon l'une quelconque des revendications 1 à 3, **caractérisé en ce que** la partie de base et la partie de couvercle sont recouvertes d'éponges en cellulose avec un renfort par fibres naturelles.

5. Dispositif selon l'une quelconque des revendications 1 à 4, **caractérisé en ce que** la partie de couvercle et/ou la partie de base sont munies de lèvres d'étanchéité.
